# EUROPÄISCHE PATENTSCHRIFT

(11) **EP 4 265 108 B1**
(45) Veröffentlichungstag und Bekanntmachung des Hinweises auf die Patenterteilung: **11.12.2024**
(21) Anmeldenummer: 23169241.9
(22) Anmeldetag: 21.04.2023
(51) Int. Cl.: A01K 67/033

(54) **ANLAGE UND VERFAHREN ZUM VERARBEITEN VON ORGANISCHEM MATERIAL**
PLANT AND METHOD FOR PROCESSING ORGANIC MATERIAL
INSTALLATION ET PROCÉDÉ DE TRAITEMENT DE MATIÈRE ORGANIQUE

(30) Priorität: 22.04.2022 DE 102022109706
(43) Veröffentlichungstag der Anmeldung: 25.10.2023
(73) Patentinhaber: Kaesemann, Thomas, 87545 Burgberg (DE)
(72) Erfinder: Kaesemann, Thomas, 87545 Burgberg (DE)
(74) Vertreter: Kimpfbeck, Thomas

(56) Entgegenhaltungen:
- CN-A- 115 644 145
- KR-B1- 102 013 244

## Beschreibung

### TECHNISCHES GEBIET

Die vorliegende Erfindung betrifft eine Anlage zur Verarbeitung von organischem Material und ein Verfahren zum Verarbeiten desselben.

### STAND DER TECHNIK

Der natürliche Vorgang von Lebenszyklen und Ernährung von Insekten/-Larven benötigt eine bestimmte Temperatur, um optimal das Wachstum und die Verwertung von Nahrung zu gewährleisten.

Bei einer industriellen Nutzung, z.B. der Gewinnung von den eigentlichen Larven als Futtermittel, bzw. Produkte aus diesen und deren Nebenprodukte, bei der organisches Ausgangsmaterial, beispielsweise sog. Biomüll oder Abfallprodukte aus der Lebensmittelherstellung, muss diese Temperatur durch Heizen oder Kühlen eingestellt werden. Dadurch entstehen Kosten durch die notwendigen technischen Komponenten und deren Instandhaltung im Betrieb sowie deren Energieverbrauch, die zu minimieren sind.

Bislang bekannte Verfahren verwenden einen diskontinuierlichen Verarbeitungsablauf bzw. Batch-Betrieb, bei dem feste Mengen Ausgangsstoff mit sog. Junglarven versetzt werden und dann bis zum Erreichen des gewünschten Wachstumsstadiums/Verwertung der Ausgangsstoffe in den notwendigen Umgebungsbedingungen gehalten werden. Dies sind bestimmt durch den Lebenszyklus der Insekten typischerweise 10 bis 14 Tage. Ein derartiges Verfahren benötigt Stell- und Verkehrsflächen. Eine Skalierung dieser bekannten Verfahren würde in gleichem Maße Personalkosten und Stellflächen erhöhen.

Anders gesagt behindert solch ein diskontinuierliches Verfahren eine Optimierung von Leistung und Wirtschaftlichkeit des Verfahrens. Durch eine energieoptimierte Auslegung des Prozesses auf eine kontinuierliche Arbeitsweise können diese Nachteile deutlich verringert und der Nutzen solcher Anlagen erheblich vergrößert werden.

CN112996382A beschreibt eine Anlage zur Insektenzucht. Dort ist eine industrielle Anlage aus multiplen, identischen Etagenbatterien mit vertikaler Ausrichtung beschrieben.

KR102013244B1 beschreibt ein modularisiertes System für die Massenaufzucht von Insekten, das in der Lage ist, Insekten in großen Mengen mittels einer nicht angetriebene Art und Weise zu produzieren, während es Insekten aufzieht, und das so auslegt ist, dass es die Arbeit erleichtert. Das modularisierte System für die Massenaufzucht von Insekten umfasst: eine Aufzuchtkammer, die belüftet, gekühlt und geheizt werden kann; und eine Aufzuchtvorrichtung, die in der Aufzuchtkammer angeordnet ist und so ausgelegt ist, dass sie Insekten in großen Mengen aufnehmen und aufziehen kann.

### AUFGABE

Somit besteht die Aufgabe der Erfindung darin eine verbesserte, leichter skalierbare, automatischere Vorrichtung zur Verarbeitung von organischem Material mittels Insektenlarven zu schaffen.

### OFFENBARUNG DER ERFINDUNG

Erfindungsgemäß wird eine Anlage zur Verarbeitung von organischem Material mit Insektenlarven gemäß Anspruch 1 vorgeschlagen. Diese Anlage umfasst eine Schienenanordnung mit einem Gefälle, mehrere Behälter, die an der Schienenanordnung verfahrbar angeordnet sind, und eine Hülle, die die Schienenanordung und die mehreren Behälter umgibt, wobei die Anlage derart ausgelegt ist, dass die Behälter unterstützt durch das Gefälle bzw. die Schwerkraft verfahren. Die Anlage umfasst ferner ein Turbinenrad, das von einem in der Hülle strömenden Fluid antreibbar ist, und einen Generator, der von dem Turbinenrad angetrieben wird, wobei das Turbinenrad und der Generator als Ventilator und Antriebsmotor betreibbar sind.

Die Schienenanordnung ist vorzugsweise aus Metall hergestellt. Die Schienenanordnung weist inbesondere eine tragende Funktion für die Anlage auf. Alternativ kann die Schienenanordnung ein Tragseil umfassen oder als Tragseil ausgebildet sein. Die Behälter können seilbahnartig entlang des Tragseils geführt werden. Dies hat den Vorteil, dass für die Hülle ein nicht-tragendes Material, wie etwa recycelter Kunststoff, eingesetzt werden kann. Das Tragseil kann Kupplungsvorrichtungen, insbesondere in äquidistantem Abstand, umfassen. Eine Kupplungsvorrichtung ist ausgelegt ein Seilgeschirr lösbar zu halten. Das Seilgeschirr ist vorzugsweise an einem der Behälter angeordnet.

Alternativ kann die Schienenanordnung eine Rutsche umfassen oder als Rutsche ausgebildet sein. Die Behälter können auf der Rutsche unterstützt durch das Gefälle der Rutsche nach unten rutschen.

Vorzugsweise weisen die Behälter polygonal angeordnete Seitenwände auf. Während des Rutschens kann so automatisch eine dichte Packung bzw. Aneinanderreihung der Behälter entstehen. Die Seitenwände der Behälter sind insbesondere hexagonal. Anders gesagt die Behälter können wabenförmig sein.

Die Schienenanordnung hat vorzugsweise eine Neigung bzw. ein Gefälle von 2 bis 12°. Insbesondere beträgt das Gefälle 5°.

Die Hülle kann ein dichtes Gehäuse für die Anlage erzielen. Das dichte Gehäuse kann die Nutzung von Abwärme und/oder Ventilation in der Anlage verbessern.

Das organische Material umfasst insbesondere pflanzliche und/oder tierische Stoffe, wie etwa Biomüll, Abfallprodukte aus der Lebensmittelherstellung, Schlachtabfälle, Grünschnitt, Klärschlamm, Trester, Zuckerrübenschnitzel, Algen, Melasse, Leinöl etc. Werden die Behälter mit Insektenlarven bzw. Junglarven und dem organischen Material beschickt, so kann letzteres als Futter für die Insektenlarven dienen. Als Insektenlarven kommen vorzugsweise Junglarven zum Einsatz. Junglarven sind insbesondere Larven, die bereits aus einem Insektenei geschlüpft sind. Die Insektenlarven reifen z.B. über eine Zeit von 10 bis 14 Tagen zu erwachsenen Larven heran, während sie das organische Material fressen. Der Eiweisgehalt der Insektenlarven erhöht sich bis zu einem Schwellwert. Anders gesagt, in den Behältern kann sich eine Insektenlarve bzw. Junglarve bis zum Erwachsenenstadium entwickeln. Ist der Eiweis-Schwellwert erreicht kann hochwertiges Eiweis bzw. Protein geernet werden, wobei das organische Material weitgehend verarbeitet bzw. entsorgt wurde. Das Protein kann z.B. als Fisch- oder allgemein als Tierfutter verwendet werden.

Zu den Larven zählen allgemein Maden und Raupen. Für die vorliegende Erfindung eignen sich z.B. Maden, insbesondere die Larven bzw. Junglarven von Waffenfliegen. Noch geeigneter sind die Larven der schwarzen Soldatenfliege, auch Hermetia Illucens genannt. Hermetia Illucens kann 2 kg Futter in 1 kg Körpermasse umwandeln. Hermetia IlIucens enthält dabei ca. 58% Protein.

Die Behälter können jeweils eine Nivellierungsvorrichtung umfassen. Die Nivellierungsvorrichtung bewirkt insbesondere einen waagerechten Behälterboden. Für ein gleichmässiges und effizientes Verarbeiten des organischen Materials kann es erforderlich sein, dass eine Schichtdicke des organischen Materials entlang des Behälterbodens möglichst konstant ist. Bei einer Schräglage des Behälterbodens bzw. eines Behälters könnte organisches Material auf eine Seite rutschen und dadurch eine unvorteilhaft unterschiedliche Schichtdicke des organischen Materials entstehen. Die Nivellierungsvorrichtung kann das Seilgeschirr umfassen, das die Behälter jeweils an dem Tragseil aufhängt. Das Seilgeschirr kann eine zentral angeordnete Umlenkrolle umfassen, sodass sich die Behälter jeweils selbst waagerecht ausrichten. Die Nivellierungsvorrichtung kann eine kardanische Aufhängung und einen Innenbehälter umfassen, die im oder am Behälter angeordnet sind. Ist der Behälter selbst geneigt, so sorgt die kardanische Aufhängung für eine Nivellierung des Innenbehälters. Ferner kann die Nivellierungsvorrichtung einen Keil umfassen, der unterhalb des Behälters anordnet ist und der ausgelegt ist das Gefälle der Schienenanordnung so auszugleichen, dass der Behälter nivelliert ist. Herkömmliche Vorrichtungen, die organisches Material lose auf Rutschen oder Bändern etc. ohne Behälter fördern sind schlechter geeignet, da durch das lose Fördern die Schichtdicke des organischen Materials stark variiert und so eine ungleichmäßige und uneffiziente Verarbeitung des organischen Materials entsteht.

Die Behälter können Stapelbehälter umfassen. Anders gesagt ein erster Behälter kann auf einen zweiten Behälter gestapelt angeordnet sein. Dadurch können mehrere der Behälter übereinander gestapelt werden.

Eine bevorzugte Schichtdicke des organischen Materials mit Larven beträgt z.B. 5 - 10 cm, noch bevorzugter ca. 8 cm. Die Behälter sind insbesondere nach oben geöffnete Gefässe mit einem ebenen Boden und rechtwinklig am Boden angeordneten Seitenwänden. Die Seitenwände sind vorzugsweise 1 bis 2 cm höher ausgelegt als die Schichtdicke des organischen Materials mit Larven. Insbesondere weisen die Behälter eine Höhe von ca. 10 cm auf. Mehrere Behälter können übereinandergestapelt sein und entlang der Schienenanordnung gefördert werden bzw. verfahren. Vorzugsweise können 2, 3, 4 oder 5 Behälter übereinander gestapelt sein. um einen effizienten Durchsatz durch die Anlage zu ermöglichen.

Während dem Heranreifen der Insektenlarven entsteht Wärmeenergie. Gemäß einer weiteren Ausbildung der Erfindung umfasst die erfindunsgemäße Anlage vorzugsweise einen Ventilator. Der Ventilator kann zur gleichmässigen Verteilung erwärmter Luft bzw. Fluid in der Hülle eingesetzt werden. Insbesondere erzeugen adultere Insektenlarven in den Behältern eine höhere Abwärme als jüngere Insektenlarven. Der Ventilator kann die Abwärme gleichmässiger in der Hülle verteilen. Dadurch kann vorteilhaft Energie gespart werden.

Desweiteren kann die Schienenanordnung spiralförmig ausgebildet sein oder spiralförmige Abschnitte aufweisen. Ferner kann die Schienenanordnung gerade Abschnitt aufweisen. Alternativ kann die Schienenanordnung spiralförmige und gerade Abschnitte aufweisen. Die spiralförmigen Abschnitte erzielen insbesondere das Gefälle. Die geraden Abschnitte können die Anlage größenskalierbar auslegen. Anders gesagt, die Anlage kann durch gerade Abschnitte ovaler bzw. längsausgedehnter ausgelegt werden, um mehr Behälter fördern zu können. Die Anlage ist so skalierbarer als herkömmliche Anlagen. Die Zeitdauer zum Führen der Behälter in der Hülle hängt insbesondere von der Verfahrstrecke für die Behälter entlang der Schienenanordnung ab. Die Zeitdauer zum Führen der Behälter in der Hülle kann von der Anzahl und/oder der Größe der Behälter in der Anlage abhängen.

In weiteren Ausgestaltung kann die Hülle zylinder- oder ovalzylinderförmig sein. Je nach verfügbarer Stellfläche für die Anlage, kann die Hülle zylinderförmig oder ovalzylinderförmig ausgelegt sein. Die Form der Hülle kann passend zur Form der Schienenanordnung ausgelegt sein. Wenn die Schienenanordnung eine tragende Funktion aufweist, so kann die Hülle aus einem nichttragenden Material gefertigt sein, wobei die Hülle an der tragenden Schienenanordnung angeordnet sein kann.

Gemäß einer Weiterbildung kann die Hülle eine erste Öffnung zum Beschicken mit Behältern und eine zweite Öffnung zum Entnehmen der Behälter umfassen. Die Behälter sind insbesondere abnehmbar an der Schienenanordnung angeordnet. Die Behälter umfassen Metall oder einen beständigen Kunststoff. Die Behälter können wiederverwendbar ausgelegt sein. Da mehrere Behälter in der Anlage gleichzeitig gefördert werden, liegt insbesondere eine kontinuierliches Verfahren vor. Herkömmliche Prozesse sind sequentiell, d.h. Larven reifen in einem Bioreaktor heran. Der Bioreaktor kann erst wieder mit neuen Larven und organischem Material beschickt werden, wenn die Larven einer vorherigen Bioreaktor-Beschickung das Erwachsenenstadium erreicht haben. Das ist im Allgemeinen diskontiuierlich, manuell aufwendig und schlecht skalierbar. Die erfindunsgemäße Anlage ermöglicht dagegen einen kontinuierlichen Eintrag mit frisch befüllten Behältern und einen kontinuierlichen Austrag mit Behältern, die erwachsene Larven und weitgehend aufgefressenes organisches Material aufweisen.

Die erfindungsgemäße Anlage umfasst das Turbinenrad, das von einem in der Hülle strömenden Fluid antreibbar ist. Von den Larven hervorgerufene Abwärme kann das strömen des Fluids bzw. der Luft in der Hülle verursachen. Die Larven können in den Behältern eine Temperatur von über 50°C hervorrufen. Steigt die Temperatur zu hoch an, sterben die Larven. Optimaler ist jedoch eine Zieltemperatur von 27°C bis 30°C. Die Strömung kann vorteilhaft zum Antrieb des Turbinenrades verwendet werden. Desweiteren umfasst die Anlage einen Generator, der von dem Turbinenrad angetrieben wird. Der Generator kann elektrische Energie erzeugen. Die Anlage ist dadurch insbesondere umweltfreundlich bzw. nachhaltig. In einem Temperaturbereich von z.B. 12°C bis 16°C sind die Larven inaktiv, d.h. sie fressen nicht. Herkömmliche Verfahren erfordern eine zusätzliche Heizung, um die Larven zunächst auf eine wachstumsförderliche Temperatur zu bringen. Die erfindungsgemäße Anlage erfordert so eine Larven-Heizung vorzugsweise nicht.

Das Turbinenrad und der Generator sind ferner als Ventilator und Antriebsmotor betreibbar. Das Turbinenrad kann den Ventilator bilden. Der Generator kann umgekehrt als Antriebsmotor geschaltet werden und den Ventilator antreiben. Ferner kann die Anlage eine Prozessteuerung umfassen, die den Antriebsmotor bzw. Generator steuert. Die Prozessteuerung kann in Abhängigkeit von der Temperatur in der Hülle steuern. Die Prozesssteuerung kann mittels mehrer Temperatursensoren die Temperatur an verschiedenen Stellen in der Hülle ermitteln. Noch bevorzugter kann die Prozesssteuerung mittels mehrerer Temperatursensoren die Temperatur von Behältern mit Larven in unterschiedlichen Wachstumsphasen ermitteln. Insbesondere erzeugen erwachsenere Insektenlarven in den Behältern eine höhere Abwärme als Junglarven.

Gemäß einer Weiterbildung der Erfindung kann die Anlage einen Wärmetauscher umfassen, der zur Aufnahme von Wärmeenergie dienen kann, die in der Anlage entsteht.

In einer weiteren Ausgestaltung kann die erfindungsgemäße Anlage eine Stetigförderungsvorrichtung zur Förderung der Behälter entlang der Schienenanordnung umfassen. Die Stetigförderungsvorrichtung kann mechanische Förderer, Schwerkraftförderer und/oder Strömungsförderer umfassen. Ein mechanischer Förderer kann einen Rollenförderer, einen Schwingungsförderer, einen Bandförderer und/oder einen Kettenförderer umfassen. Ein Schwerkraftförderer kann einen Rollenbahn, Kugelbahn und/Schienenbahn umfassen. Ein Strömungsförderer kann eine pneumatische oder eine hydraulische Förderung umfassen. Stetigförderungsvorrichtung sind meist kompakt und einfach konstruiert, erfordern wenig Wartung, wenig manuelle Bedienung, da automatisch arbeitend, und eignen sich besonders, um die Behälter entlang der schwach geneigten Schienenanordnung zu fördern. Außerdem kann die Stetigförderungsvorrichtung die Haftreibung der Behälter an der Schienenanordnung überwinden, um so eine Förderung der Behälter entlang des Gefälles der Schienenanordnung zu unterstützen. Anders gesagt die Stetigförderungvorrichtung und das Gefälle der Schienenanordnung bewirken in Kombination eine Förderung der Behälter entlang der Schienenanordnung.

Ferner zeichnet sich die Erfindung aus durch ein erfindungsgemäßes Verfahren zum Verarbeiten von organischem Material mittels der erfindungsgemäßen Anlage. Das Verfahren umfasst folgende Schritte: Vermischen von organischem Material, insbesondere organische Abfälle, mit Insektenlarven; Befüllen eines Behälters mit dem Gemisch; Führen des Behälters entlang einer Schienenanordnung in einer die Schienenanordnung umgebenden Hülle unterstützt durch Schwerkraft, wobei der Behälter für eine Zeitdauer in der Hülle geführt bzw. gefördert wird, die von einer Umwandlungsgeschwindigkeit des organischen Material von den Insektenlarven in ein Umwandlungsprodukt bzw. von einem Gehalt des Umwandlungsprodukts abhängt.

Herkömmliche Verfahren sind manuell aufwendig, während bei dem erfindungsgemäßen Verfahren der Behälter im Wesentlichen Schwerkraftunterstützt bewegt wird. Die Geschwindigkeit des Verfahren kann durch die Anordnung und Größe von Behälter und Schienenanordnung beeinflusst werden. Insbesondere erzeugen die Insektenlarven mit einer fortschreitenden Umwandlung des organischen Materials in das Umwandlungsprodukt Wärmeenergie. Das Umwandlungsprodukt umfasst im Wesentlichen Eiweiß bzw. Protein der Insektenlarven selbst. Die Wärmeenergie kann ein Fluid, insbesonder Luft, in der Hülle erhitzen, wobei das Fluid in der Hülle strömt.

Vorteilhafte Weiterbildungen der Erfindung sind in den Unteransprüchen angegeben und in der Beschreibung beschrieben.

### KURZBESCHREIBUNG DER ZEICHNUNGEN

Ausführungsbeispiele der Erfindung werden anhand der Zeichnungen und der nachfolgenden Beschreibung näher erläutert. Es zeigen:
- Figur 1a: eine Anlage gemäß eines Ausführungsbeispiels der Erfindung,
- Figur 1b: eine weitere Anlage gemäß eines Ausführungsbeispiels der Erfindung,
- Figur 2a: eine weitere Anlage gemäß eines Ausführungsbeispiels der Erfindung,
- Figur 2b: eine weitere Anlage gemäß eines Ausführungsbeispiels der Erfindung,
- Figur 3: einen Behälter gemäß eines Ausführungsbeispiels der Erfindung,
- Figur 4a: eine Schienenanordnung gemäß eines Ausführungsbeispiels der Erfindung und
- Figur 4b: eine weitere Anlage gemäß eines Ausführungsbeispiels der Erfindung.

### AUSFÜHRUNGSFORMEN DER ERFINDUNG

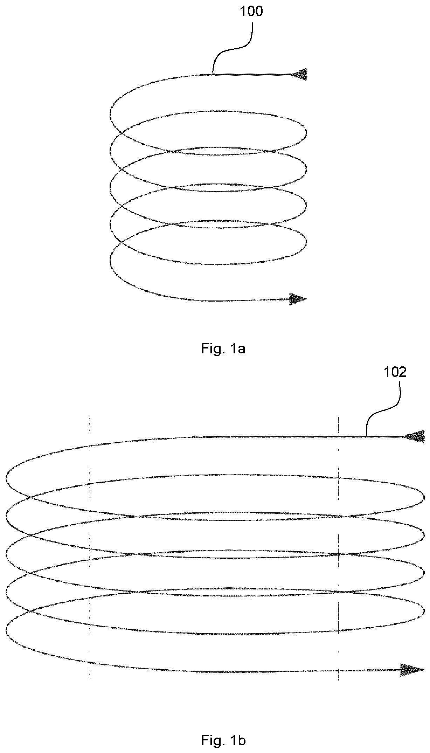

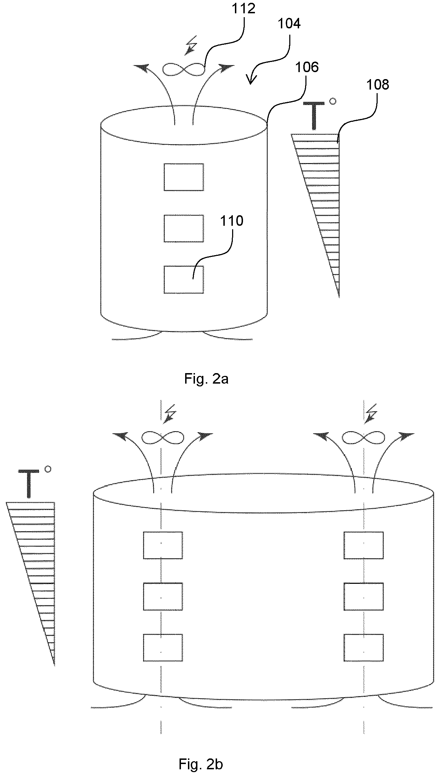

Die Aufgabe der Erfindung wird dadurch gelöst, dass Materialfluss und Kühl- bzw. Warmluft als Gegenstromverfahren dargestellt werden. Um einen kontinuierlichen Materialfluss zu erreichen, wird das zu verarbeitende, überwiegend organische Material in geeigneten Behältern in einer Spiralführung, Fig. 1a und 1b auf Leitschienen (Schienenanordnung) schwerkraftunterstützt über den für die Erreichung des Prozessergebnisses notwendigen Zeitraum von der Materialaufgabe "oben" zur Materialausleerung "unten" bewegt. Der Zylinderaufbau erfordert Materialbehälter, die einen identischen Rauminhalt haben, aber gleichzeitig eine feste Materialschichtdicke garantieren müssen. Dies wird als eine Variante durch unterschiedliche Grundflächen erreicht, je nach Entfernung von der Rotationsmitte des Anlagenkörpers. Gedehnte Zylinder erfordern eine Anpassung der Behälterlagerung/-führung für die Bewegung über gerade Abschnitte. Solche gedehnten Zylinder ermöglichen eine größere Materialmenge, die den Prozess durchlaufen und vermeiden, lediglich die Anzahl der Zylinder zur Erhöhung des Durchsatzes zu steigern. Bei optimaler Regelung ist die Eigenprozesswärme ausreichend, um ohne Fremdenergiezugabe einen hohen Wirkungsgrad zu erreichen. Die Regelung erfolgt durch individuell ansteuerbare Schieber in der Außenverkleidung der Anlage schematisch in Fig. 2a und 2b. Durch die entstehende Aufwärtsbewegung des Luftstromes innerhalb der Anlage ist je nach Betriebszustand eine energetische Rückgewinnung durch mechanische Nutzung z.B. über ein Generatorrad und/oder der im Prozess nicht aufgenommenen Wärmemenge möglich. Im Detail betrachtet erlaubt die räumliche Führung der Materialbehälter einen hohen Grad an Automatisierung bei Entleerung, Reinigung und Befüllung. Hierzu ist es möglich, durch spiralförmiges Verlegen der Führungsschienen der Materialbehälter in Bewegungsrichtung die notwendigen Bewegungen ohne manuelles Eingreifen auszuführen. Der Aufbau der Anlage kann in einem erheblichen Grad aus recycelten Materialien angefertigt werden (insbesondere, aber nicht nur, ist dabei die statisch nicht tragende Außenhülle gemeint, die aus nicht sortenreinem Altplastik - z.B. aus aus der Umwelt/Meeren zurückgeführtem Wertstoff- mit wärmedämmenden Eigenschaften aufgebaut werden kann). Die Anlage kommt vorzugsweise ohne Antrieb für die Behälter aus. Die Anlage kann einen Ventilator zur Umwältzung in der Hülle umfassen. Sollte in Abhängigkeit der Schienenneigung der resultierende Vektor der Schwerkraft nicht ausreichend sein, die Behälter in Prozessrichtung voran zu bewegen, können Systeme der Vibrationsförderung oder andere Systeme eines Antriebs mit geringem Energieverbrauch eingesetzt werden.

Figur 1a zeigt einen Materialfluss 100. Eine Schienenanordnung mit mehreren daran beweglich angeordneten Behältern bewirkt den Materialfluss 100. Das Material umfasst Larven von Hermetia Illucens sowie pflanzliche und tierische Abfallstoffe. Die Behälter werden mit dem Material beschickt und schwerkraftunterstützt im Wesentlichen selbsttätig entlang des Materialflusses 100 gefördert.

Figur 1b zeigt einen Materialfluss 102, der ovalzylindrisch geformt ist. Die ovale Form des Materialflusses 102 ermöglicht es, mehr Behälter zu fördern als in Materialfluss 100.

Figur 2a zeigt eine Anlage 104 zur Verarbeitung von pflanzliche und tierische Abfallstoffen. Die Anlage 104 umfasst eine dichte Hülle 106. In der Hülle 106 werden mehrere Behälter entlang eines spiralförmigen Weges gefördert. Der spiralförmige Weg hat ein Gefälle, sodass die Behälter schwerkraftunterstützt entlang des Weges gleiten. Die Behälter werden mit Junglarven von Hermetia Illucens und organischem Material beschickt, das als Futter für die Junglarven dient. Die Junglarven reifen z.B. über eine Zeit von 10 bis 14 Tagen heran, wobei sich ihr Eiweisgehalt bis zu einem Schwellwert erhöht. Anders gesagt, in den Behältern kann sich eine Junglarve bis zum Erwachsenenstadium entwickeln. Während dem Heranreifen der Larven entsteht Wärmeenergie. Die Wärmeenergie erhitzt Luft in der Hülle, die nach oben strömt und einen oberen Bereich des Behälters erhitzt, veranschaulicht durch Temperaturgradient 108. Die Hülle 106 wirkt dabei wie ein Kamin. Die Anlage 104 umfasst ferner mehrere Belüftungsklappen 110. Eine Prozesssteuerung ermittelt mittels mehrerer Temperatursensoren die Temperatur an verschiedenen Stellen in der Hülle 106 und steuert die Belüftungsklappen 110, um eine vorbestimmte Temperatur, z.B. 27°C bis 30°C in einem vorbestimmten Bereich der Hülle 106 herstellen bzw. konstant halten zu können. Eine Turbine-Generator-Anlage 112 die auch als Ventilator-Antriebsmotor-Anlage 112 betrieben werden kann, wird von der Prozessteuerung derart angesteuert, dass eine vorbestimmte Temperatur in einem vorbestimmten Bereich der Hülle 106 herstellt bzw. konstant gehalten werden kann.

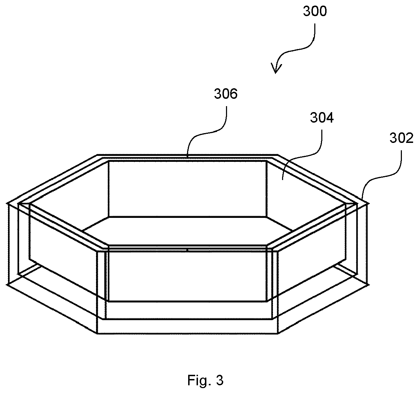

Figur 3 zeigt einen wagenförmigen Behälter 300. Der Behälter 300 umfasst einen Außenbehälter 302 mit einem Boden und sechs Seitenwänden. Die Seitenwände sind hexagonal am Boden angeordnet, sodass die Wabenform entsteht. Ferner umfasst der Behälter 300 einen Innenbehälter 304 und eine kardanische Aufhängung 306, die den Innenbehälter kardanisch bezüglich des Außenbehälter 302 lagert. Der Innenbehälter 304 weist etwas kleinere Abmessungen auf als die Innenabmessungen des Außenbehälters 302. In horizonaler Lage ist der Innenbehälter bündig im Außenbehälter angeordnet bzw. versenkt. Wenn der Behälter 300 entlang eines Gefälles gefördert bzw. verfahren wird, so neigt sich der Innenbehälter 304 derart, dass das Gefälle ausgeglichen bzw. nivelliert wird. Für ein gleichmässiges und effizientes Verarbeiten von organischem Material mit Larven in dem Behälter 300 ist es erforderlich, dass eine Schichtdicke des organischen Materials entlang des Bodens möglichst konstant ist. Während des Rutschens des Behälters 300 entlang einer Rutsche sorgt die wabenförmige Form des Behälters 300 automatisch für ein dichte Packung bzw. Aneinanderreihung zu anderen wabenförmigen Behältern auf der Rutsche.

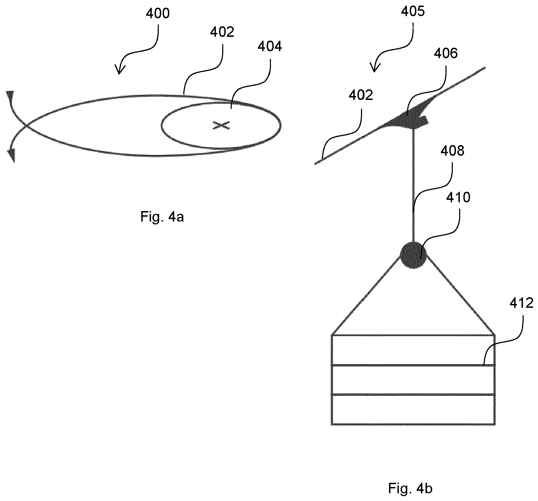

Figur 4a zeigt eine Schienenanordnung 400 mit einem Tragseil 402 und einer Umlenkrolle 404. Das Tragseil 402 verläuft spiralförmig von oben nach unten (durch Pfeile angezeigt).

Figur 4b zeigt eine Anlage 405 mit dem Tragseil 402, einer Seilkupplungsvorrichtung 406, einem Seilgeschirr 408, einer Nivellierungsvorrichtung 410 und mehreren gestapelten Behältern 412. Das Tragseil 402 hat ein Gefälle von 5° zum Erdboden. Am Tragseil 402 sind die Seilkupplungen 406 äquidistant angeordnet. Die Seilkupplungen 406 halten jeweils ein Seilgeschirr 408 lösbar.

Am Seilgeschirr sind mehrere gestapelte Behälter 412 aufgehängt. Das Seilgeschirr 408 umfasst eine Nivellierungsvorrichtung 410, die z.B. als Rolle ausgelegt sein kann. Während des Förderns der gestapelten Behälter 412 sind die Behälter durch die Nivellierungsvorrichtung 410 eben bzw. waagerecht ausgerichtet. In den Behältern wird organisches Material, das mit Larven der schwarzen Soldatenfliege durchsetzt ist, befördert. Das Material wird während der Beförderung von den Larven gefressen, d.h. verarbeitet. Für eine gleichmässige Verarbeitung ist eine ebene Ausrichtung bzw. Nivellierung der Behälter erforderlich. Das Seilgeschirr 408 und die gestapelten Behälter 412 werden in der Anlage oben in die Seilkupplungsvorrichtung 406 eingekoppelt und durch die Anlage langsam über 10 bis 14 Tage gefördert. Nach diesem Zeitraum werden das Seilgeschirr 408 und die gestapelten Behälter 412 in der Anlage 405 unten ausgekoppelt.

### BEZUGSZEICHENLISTE

- 100: Materialfluss
- 102: Materialfluss
- 104: Anlage
- 106: Hülle
- 108: Temperaturgradient
- 110: Belüftungsklappen
- 112: Turbine-Generator-Anlage
- 300: Behälter
- 302: Außenbehälter
- 304: Innenbehälter
- 306: kardanische Aufhängung
- 400: Schienenanordnung
- 402: Tragseil
- 404: Umlenkrolle
- 405: Anlage
- 406: Seilkupplungsvorrichtung
- 408: Seilgeschirr
- 410: Nivellierungsvorrichtung
- 412: Behälter

## Patentansprüche

1. Anlage (104; 405) zur Verarbeitung von organischem Material mit Insektenlarven mit:
einer Schienenanordnung (400, 402) mit einem Gefälle,
mehreren Behältern (300, 302, 304; 412), die an der Schienenanordnung (400, 402) verfahrbar angeordnet sind, und
einer Hülle (106), die die Schienenanordung (400, 402) und die mehreren Behälter (300, 302, 304; 412) umgibt,
wobei die Anlage (140; 405) derart ausgelegt ist, dass die Behälter (300, 302, 304; 412) unterstützt durch das Gefälle verfahren
**dadurch gekennzeichnet, dass**
die Anlage ein Turbinenrad (112), das von einem in der Hülle strömenden Fluid antreibbar ist, und
einen Generator (112), der von dem Turbinenrad angetrieben wird, umfasst,
wobei das Turbinenrad (112) und der Generator (112) als Ventilator und Antriebsmotor betreibbar sind.

2. Anlage (104; 405) nach Anspruch 1
mit einem Ventilator.

3. Anlage (104; 405) nach einem der vorhergehenden Ansprüche
wobei die Schienenanordnung (400, 402) spiralförmige Abschnitte aufweist.

4. Anlage (104; 405) nach einem der vorhergehenden Ansprüche
wobei die Hülle (106) zylinder- oder ovalzylinderförmig ist.

5. Anlage (104; 405) nach einem der vorhergehenden Ansprüche
wobei die Hülle (106) eine erste Öffnung zum Beschicken mit Behältern (300, 302, 304; 412) und eine zweite Öffnung zum Entnehmen der Behälter (300, 302, 304; 412) umfasst.

6. Anlage (104; 405) nach einem der vorhergehenden Ansprüche
mit einem Wärmetauscher, zur Aufnahme von Wärmeenergie, die in der Anlage (104; 405) entsteht.

7. Anlage (104; 405) nach einem der vorhergehenden Ansprüche
mit einer Stetigförderungsvorrichtung zur Förderung der Behälter entlang der Schienenanordnung (400, 402).

8. Anlagen (104; 405) nach einem der vorhergehenden Ansprüche,
wobei die Behälter (300, 302, 304; 412) jeweils eine Nivellierungsvorrichtung (410) umfassen.

9. Verfahren zum Verarbeiten von organischem Material mittels einer Anlage gemäß einem der vorhergehenden Ansprüche, umfassend die Schritte:
Vermischen von organischem Material, insbesondere organische Abfälle, mit Insektenlarven;
Befüllen eines Behälters mit dem Gemisch;
Führen des Behälters entlang einer Schienenanordnung in einer die Schienenanordnung umgebenden Hülle unterstützt durch Schwerkraft, wobei der Behälter für eine Zeitdauer in der Hülle geführt wird, die von einer Umwandlungsgeschwindigkeit des organischen Material von den Insektenlarven in ein Umwandlungsprodukt bzw. von einem Gehalt des Umwandlungsprodukts abhängt.

10. Verfahren nach Anspruch 9,
wobei die Insektenlarven mit einer fortschreitenden Umwandlung des organischen Materials in das Umwandlungsprodukt Wärmeenergie erzeugen.

11. Verfahren nach Anspruch 9, wobei die Wärmeenergie ein Fluid in der Hülle erhitzt, das in der Hülle strömt, insbesondere aufsteigt.

12. Verfahren nach einem der Ansprüche 9 bis 11, wobei das Umwandlungsprodukt Eiweiß umfasst.

## Claims

1. System (104; 405) for processing organic material with insect larvae, comprising:
a rail arrangement (400, 402) which has a gradient,
a plurality of containers (300, 302, 304; 412) which are arranged so as to be movable on the rail arrangement (400, 402), and
a shell (106) which surrounds the rail arrangement (400, 402) and the plurality of containers (300, 302, 304; 412),
the system (140; 405) being designed such that the containers (300, 302, 304; 412) move in a manner assisted by the gradient
**characterized in that**
the system comprises a turbine wheel (112) which is drivable by a fluid which flows in the shell, and
a generator (112) which is driven by the turbine wheel,
the turbine wheel (112) and the generator (112) being operable as a fan and drive motor.

2. System (104; 405) according to claim 1
comprising a fan.

3. System (104; 405) according to any of the preceding claims
wherein the rail arrangement (400, 402) has spiral-shaped portions.

4. System (104; 405) according to any of the preceding claims
wherein the shell (106) is cylinder-shaped or oval-cylinder-shaped.

5. System (104; 405) according to any of the preceding claims
wherein the shell (106) comprises a first opening for loading with containers (300, 302, 304; 412), and a second opening for removing the containers (300, 302, 304; 412).

6. System (104; 405) according to any of the preceding claims
comprising a heat exchanger for absorbing thermal energy which is produced in the system (104; 405).

7. System (104; 405) according to any of the preceding claims
comprising a continuous conveying device for conveying the containers along the rail arrangement (400, 402).

8. System (104; 405) according to any of the preceding claims,
wherein the containers (300, 302, 304; 412) each comprise a leveling device (410).

9. Method for processing organic material by means of a system according to any of the preceding claims, comprising the steps of:
mixing organic material, in particular organic waste, with insect larvae;
filling a container with the mixture;
guiding the container along a rail arrangement, in a manner assisted by gravity, in a shell which surrounds the rail arrangement, wherein the container is guided in the shell for a period of time which depends on a conversion rate of the organic material by the insect larvae into a conversion product, or on a content of the conversion product.

10. Method according to claim 9,
wherein the insect larvae generate thermal energy by means of a progressive conversion of the organic material into the conversion product.

11. Method according to claim 9, wherein the thermal energy heats a fluid in the shell, which fluid flows, in particular rises, in the shell.

12. Method according to any of claims 9 to 11, wherein the conversion product comprises protein.

## Revendications

1. Installation (104 ; 405) pour le traitement de matière organique avec des larves d'insectes comportant :
un agencement de rails (400, 402) comportant une pente,
plusieurs récipients (300, 302, 304 ; 412) qui sont disposés de manière à pouvoir se déplacer sur l'agencement de rails (400, 402), et
une enveloppe (106) qui entoure l'agencement de rails (400, 402) et les récipients (300, 302, 304 ; 412),
dans laquelle l'installation (140 ; 405) est conçue de telle sorte que les récipients (300, 302, 304 ; 412) se déplacent avec l'assistance de la pente
**caractérisée en ce que**
l'installation comprend une roue de turbine (112) qui peut être entraînée par un fluide s'écoulant dans l'enveloppe, et
un générateur (112) qui est entraîné par la roue de turbine,
dans laquelle la roue de turbine (112) et le générateur (112) peuvent fonctionner comme un ventilateur et un moteur d'entraînement.

2. Installation (104 ; 405) selon la revendication 1
comportant un ventilateur.

3. Installation (104 ; 405) selon l'une des revendications précédentes
dans laquelle l'agencement de rails (400, 402) présente des sections en forme de spirale.

4. Installation (104 ; 405) selon l'une des revendications précédentes
dans laquelle l'enveloppe (106) est en forme de cylindre ou de cylindre ovale.

5. Installation (104 ; 405) selon l'une des revendications précédentes
dans laquelle l'enveloppe (106) comprend une première ouverture permettant le chargement de récipients (300, 302, 304 ; 412) et une seconde ouverture permettant de retirer les récipients (300, 302, 304 ; 412).

6. Installation (104 ; 405) selon l'une des revendications précédentes
comportant un échangeur de chaleur, pour l'absorption d'énergie thermique produite dans l'installation (104 ; 405).

7. Installation (104 ; 405) selon l'une des revendications précédentes
comportant un dispositif de transport continu permettant de transporter les récipients le long de l'agencement de rails (400, 402).

8. Installation (104 ; 405) selon l'une des revendications précédentes,
dans laquelle les récipients (300, 302, 304 ; 412) comprennent respectivement un dispositif de mise à niveau (410).

9. Procédé pour le traitement de matière organique au moyen d'une installation conformément à l'une des revendications précédentes, comprenant les étapes consistant à :
mélanger une matière organique, en particulier des déchets organiques, avec des larves d'insectes ;
remplir un récipient du mélange ;
guider le récipient le long d'un agencement de rails dans une enveloppe entourant l'agencement de rails, avec l'assistance de la gravité, dans lequel le récipient est guidé dans l'enveloppe pendant une durée qui dépend d'une vitesse de transformation de la matière organique des larves d'insectes en un produit de transformation ou d'une teneur du produit de transformation.

10. Procédé selon la revendication 9,
dans lequel les larves d'insectes produisent de l'énergie thermique avec une transformation progressive de la matière organique en produit de transformation.

11. Procédé selon la revendication 9, dans lequel l'énergie thermique chauffe un fluide dans l'enveloppe, lequel fluide s'écoule, en particulier monte, dans l'enveloppe.

12. Procédé selon l'une des revendications 9 à 11, dans lequel le produit de transformation comprend des protéines.
